# EUROPÄISCHE PATENTSCHRIFT

(11) **EP 0 787 804 B1**
(45) Veröffentlichungstag und Bekanntmachung des Hinweises auf die Patenterteilung: **23.03.2005**
(21) Anmeldenummer: 97101265.3
(22) Anmeldetag: 28.01.1997
(51) Int. Cl.: C12N 15/23, C12P 21/02, C07K 14/57

(54) **Verfahren zur Synthese von löslichen, rekombinanten Proteinen aus Bakterienzellen**
Process for the synthesis of soluble recombinant protein from bacterial cells
Procédé de synthése de proteines recombinantes solubles à partir de cellules bactériennes

(30) Priorität: 08.02.1996 DE 19604583
(43) Veröffentlichungstag der Anmeldung: 06.08.1997
(73) Patentinhaber: Fraunhofer-Gesellschaft zur Förderung der angewandten Forschung e.V., 80686 München (DE)
(72) Erfinder: Bernd, Otto, Dr., 30659 Hannover (DE); Waschütza, Gero, Dr., 28536 Meinersen (DE); Zakaria, Hayssam, 30880 Zaatzen (DE)

(56) Entgegenhaltungen:
- EP-A- 0 300 425
- WO-A-92/08737
- SCHEIN C. ET AL.: "Formation of soluble recombinant proteins in Escherichia coli is favored by lower growth temperature" BIO/TECHNOLOGY, Bd. 6, März 1988, Seiten 291-294, XP002092249
- WATERS S. AND NEUJAHR H.: "A fermentor culture for production of recombinant phenol hydroxylase" PROTEIN EXPRESSION AND PURIFICATION, Bd. 5, 1994, Seiten 534-540, XP002092250
- MIZUKAMI T. ET AL.: "Production of active human interferon beta in E. coli" BIOTECHNOLOGY LETTERS, Bd. 8, Nr. 9, 1986, Seiten 605-610, XP002092251
- ATKINSON T. ET AL.: "Inclusion bodies or product secretion in bioprocessing" WORLD BIOTECH REPORT,1988, Seiten 399-405, XP002092252

## Beschreibung

Lösliche, rekombinante Proteine werden im Regelfall aus Bakterien hergestellt, in der Weise, daß ein Expressionsplasmid exprimiert, das Protein isoliert und gereinigt wird. Derartige Verfahren sind für die verschiedensten Proteine bekannt. Z.B wird für die Expression von Interferon-γ in E.coli das Plasmid PKK 233-2 konstruiert (E. Ammann und J. Brosius; Gene 40, 1985, 1893 bis 1900). Dieses Plasmid besitzt einen induzierbaren trc Promotor, eine multiple Cloningsite, die das Startcodon (ATG) enthält sowie Terminatoren für die RNA-Polymerase.

Ein identisches Verfahren ist aus der DE 40 36 856.4 bekannt. In diesem Dokument ist u.a. auch ein Verfahren zum Herstellen einer verkürzten Interferon-γ Variante (Interferon-γ 10L) beschrieben. Auch hierbei wird wiederum von einem entsprechenden Expressionsplasmid ausgegangen. Auch dieses Plasmid wird in Bakterienzellen (E.coli) mit einer Rate von 30 % des totalen Proteins exprimiert. Zu über 90 % liegt dabei dieses Interferon-γ in Form von unlöslichen, sog. "Inclusionbodies" vor. Für die Reinigung dieses In-Interferon-γ werden dabei die Bakterienzellen nach erfolgreicher Expression aufgebrochen und die "Inclusionbodies" durch mehrfaches Waschen von löslichen bakteriellen Proteinen befreit. Das Aufbrechen wird bevorzugt mechanisch, insbesondere durch Ultraschall, vorgenommen. Die "Inclusionbodies" werden dann durch einen Denaturierungsschritt in Lösung gebracht und nachfolgend einem Renaturierungsschritt unterzogen. Der Renaturierungsschritt ist deshalb erforderlich, weil bei der Herstellung der Proteine aus den "Inclusionbodies" das erhaltene Protein nicht in der biologisch aktiven Form vorliegt. Durch den Renaturierungsschritt soll wieder eine Faltung in die biologisch aktive Form erfolgen.

Nachteilig bei diesen Verfahren des Standes der Technik ist jedoch, daß zum einen die Ausbeute bei der Herstellung der Proteine aus den "Inclusionbodies" sehr gering ist (z.B. beim vorstehend beschriebenen Verfahren ca. 10 %) und daß es sich zum anderen gezeigt hat, daß durch den Renaturierungsschritt nicht immer gewährleistet ist, daß das Protein wieder in seine biologisch aktive Form vollständig zurückgefaltet wird. Es hat sich herausgestellt, daß bei den Proteinen, die aus den "Inclusionbodies" gewonnen werden, zumeist eine inhomogene Strukturpopulation für das Protein vorliegt.

Ausgehend hiervon ist es die Aufgabe der vorliegenden Erfindung, das vorstehend beschriebene Verfahren in der Weise zu verbessern, daß zum einen eine deutliche Steigerung der Ausbeute erreicht wird und daß das Protein möglichst in seiner aktiven Form isoliert wird, ohne daß eine Rückfaltung erforderlich ist.

Die Aufgabe wird durch die kennzeichnenden Merkmale des Anspruches 1 gelöst. Die Unteransprüche zeigen vorteilhafte Weiterbildungen auf.

Überraschenderweise hat sich gezeigt, daß es durch die genannte Einhaltung der Verfahrensparameter nach Anspruch 1 bei der Exprimierung der Plasmide möglich ist, das Protein in einer Lösung zu erhalten. Nach der Induktionsphase verbleiben 20-60 % des Proteins als lösliches Protein, nur der Rest ist in Form von "Inclusionbodies" präzipitiert. Bei den Verfahren des Standes der Technik präzipitieren zumeist mehr als 95 % des Proteins in Form von "Inclusionbodies". Der Vorteil dieses Verfahrens liegt darin, daß die Faltung des Proteins, z.B. des Interferon-γ, in die räumliche Struktur in der Bakterienzelle selbst abläuft. Bei diesen Verfahren ist somit nicht, wie bei den Verfahren des Standes der Technik, ein Denaturierungs- und Renaturierungsschritt erforderlich, da sich die räumliche Struktur in der Bakterienzelle selbst bildet. Eine unvollständige Rückfaltung ist deshalb ausgeschlossen. Besonders hervorzuheben ist bei diesem Verfahren, daß sich damit nicht nur ein Protein in der aktiven Form in homogener Weise bildet, sondern auch die Ausbeute bei diesem Verfahren, z.B. für Interferon-γ, bei 50 % im Gegensatz zu ca. 10 % bei den Verfahren des Standes der Technik liegt.

Gemäß der Erfindung wird bei den Proteinen von E. coli ausgegangen, als Promotor wird ein trc-Promotor und als Induktor ein IPTG-Induktor verwendet.

Für das erfindungsgemäße Verfahren ist es wichtig, daß die im Anspruch 1 angeführten Verfahrensmerkmale genau und exakt eingehalten werden. Nur durch die Kombination der einzelnen Verfahrensmerkmale a) bis e) ist es gewährleistet, daß die Proteine aus dem Proteinüberstand, d.h. aus einer Proteinlösung, gewonnen werden können. Wesentlich bei den Verfahrensparametern ist, daß die Zellkultur bis zu einer OD 600 nm von 0,4 bis 0,6 angezogen wird und daß dann eine Abkühlung in einer bestimmten, definierten Zeitspanne (15-40 Min.) auf eine bestimmte, definierte Temperatur (20 bis 26°C) erfolgt. Diese Abkühlung in Verbindung mit der sehr niedrigen Induktorkonzentration (10 bis 55 µM) gewährleistet, daß nach einer Induktionsphase bei 20 bis 26°C von 3,5 bis 6,5 Std. 20 bis 60 % des Proteins in löslicher Form vorliegen. der Rest präzipitiert in "Inclusionbodies".

Ausführliche Untersuchungen haben gezeigt, daß z.B. bei Temperaturen von 37°C während der Induktionsphase mehr als 95 % des Proteins zu "Inclusionbodies" präzipitieren. Offensichtlich hat somit die Temperaturabsenkung in Verbindung mit der niedrigen Induktor-Konzentration eine entscheidende Bedeutung beim erfindungsgemäßen Verfahren.

Die Isolierung und Reinigung des Proteins wird dabei so vorgenommen, daß zuerst eine Fraktionierung erfolgt. Dazu werden die Bakterienzellen nach der Expressionsphase wie bekannt aufgebrochen (z.B. durch Ultraschall) und das Zellysat mit Puffer aufgenommen. Durch Zentrifugieren kann dann ein Proteinüberstand und "Inclusionbodies" erhalten werden. Für die weitere Aufarbeitung wird nur noch dieser Proteinüberstand verwendet. Der Überstand wird dabei einer Hochreinigung unterzogen. Dies kann z.B. so durchgeführt werden, daß der Proteinüberstand mit einem Kationenaustauschermaterial gerührt wird. Dadurch erfolgt eine Bindung des Proteins an den Kationenaustauscher. Das mit Protein beladene Kationenaustauschermaterial kann mit Phosphat-Puffer gewaschen und das Protein z.B. mit Kochsalzlösung in einem Puffer eluiert werden. Zur Hochreinigung kann dann noch eine Ammoniumsulfatfällung und eine Gelfiltration angeschlossen werden.

In zahlreichen Versuchen hat es sich herausgebildet, daß es günstig ist, wenn das Verfahren unter Einhaltung folgender Parameter durchgeführt wird:
Verfahrensschritt a); OD 600 nm von 0,45 bis 0,55 bei 35 bis 38°C, besonders bevorzugt von 0,5 bei 37°C.
Verfahresschritt b); Zeitspanne 25 bis 35 Min., besonders bevorzugt 30 Min.; Abkühlung auf 24 bis 26°C, besonders bevorzugt 25°C;
Verfahrensschritt c); Zugabe von 10 bis 50 µM, besonders bevorzugt von 50 µM Induktor; und
Verfahrensschritt d); Induktionsphase bei 24 bis 26°C von 3,5 bis 5,5 Std., besonders bevorzugt bei 25°C über 5 Std.

Es hat sich gezeigt, daß das erfindungsgemäße Verfahren besonders zur Herstellung von Interferon-γ und seinen Derivaten geeignet ist und daß mit diesem Verfahren nicht nur Interferon-γ in der vorstehend beschriebenen aktiven und hochreinen Form gewonnen werden kann, sondern auch Varianten davon, die z.B. um 10 Aminosäuren verkürzt sind oder auch Varianten, die eine Disulfidbrücke aufweisen. Hier sowohl in reduzierter als auch in oxidierter Form. Aus der Literatur ist bisher noch nicht bekannt, daß aus "Inclusionbodies" Interferon-γ in reduzierter Form gewonnen werden kann. Das erfindungsgemäße Verfahren zeichnet sich somit nicht nur dadurch aus, daß Proteine mit hoher Ausbeute in reiner Form erhalten werden können, sondern dieses Verfahren ermöglicht offensichtlich auch die Exprimierung von Proteinen, wie sie bisher über das Verfahren mit den "Inclusionbodies" nicht möglich waren.

Das erfindungsgemäße Verfahren wird nachfolgend ausführlich anhand der Herstellung von Interferon-γ beschrieben. Wie vorstehend erläutert, ist das Verfahren jedoch nicht auf Interferon-γ beschränkt, sondern ist grundsätzlich auf alle löslichen Proteine, die aus Bakterienzellen exprimieren, anwendbar.

### Expression (Synthese) des IFNγ

E.coli Zellen (JM 105) mit einem Expressionsplasmid, das das komplette Gen für IFNγ hinter dem trc-Promotor enthält, werden bis zu einer OD 600 nm von 0,5 bei 37°C hochgezogen. Gestartet wurde ein 500 ml Ansatz YT Medium, der mit 5 ml einer oben genannten Übernacht-Kultur angeimpft wurde.

Die Zellkultur wird in einer 30-Minuten-Phase auf 25°C abgekühlt. Die Synthese des IFNγ wird durch die Zugabe von 50 µM IPTG Endkonzentration induziert. Die Induktionsphase wird bei einer Temperatur von 25°C über einen Zeitraum von 5 Stunden durchgeführt. Nach dieser Induktionsphase verbleiben 20 bis 60 % des IFNγ als ein lösliches Protein, der Rest des IFNγ ist in Form von "Inclusionbodies" präzipitiert.

### Fraktionierung des IFNγ

Die Bakterienzellen werden nach der 5-stündigen Expressionsphase durch Zentrifugation (15 Min.; 6000 rpm in einem JA 15 Rotor) in Form des Zellpellets gesammelt. Die Zellen werden in 200 ml PBS Puffer (Phosphat Buffered Saline) oder Na-Phosphatpuffer pH 7,0 durch Resuspendieren gewaschen. Durch erneute Zentrifugation wie oben wird ein gewaschenes Zellpellet gewonnen, das entweder gleich in der Reinigung verwertet oder bei -70°C gelagert wird.

Das gewaschene Zellpellet wird in 15 ml PBS oder Na-Phosphat-Puffer aufgenommen und die Zellen durch Ultraschall geöffnet (5 x 10 Sek. unter Kühlung auf Eis). Zum Zellysat werden 15 ml Puffer hinzugegeben und das verdünnte Zellysat durch Zentrifugation (30 Min; 10000 rpm; 7A 20 Rotor) fraktioniert zu einem löslichen Proteinüberstand (Fraktion I) und einem Zellysatpellet, das die "Inclusionbodies" enthält.

### Hochreinigung des löslichen IFNγ SP-Sepharose:

In die Fraktion I werden vorsichtig 1 ml einer im gleichen Puffer äquilibrierten SP-Sepharose (mit einer Kationenbindung von 20 mg Protein) gerührt. NAch 30 Minuten auf Eis wird 10 Minuten bei 3500 rpm zentrifugiert. Das SP-Sepharosepellet, an das IFNγ gebunden ist, wird in 2 sukzessiven Waschschritten mit je 15 ml Puffer von E.coli Proteinen befreit. Das gebundene IFNγ wird mit 2 ml 1 M NaCl voll im Puffer eluiert und durch Zentrifugation vom SP-Sepharosematerial abgetrennt. Die Elutionsschritte werden zweimal wiederholt, bis sichergestellt ist, daß alles IFNγ vom SP-Sepharosematerial eluiert wurde. Die Elution des Proteins wurde durch eine OD 280/260 mm-Messung verfolgt. Die beiden Fraktionen mit der höchsten OD, normalerweise die beiden ersten Eluate, werden zur Fraktion II vereinigt. Dieser Reinigungsschritt ist wegen des hohen IP (isoelektrischer Punkt des IFNγ von mehr als 10) sehr effizient. In diesem Batchverfahren wird die Reinheit des IFNγ von ungefähr 5 % auf mehr als 90 % erhöht.

### Ammoniumsulfat-Füllung

Fraktion II (4 ml) wird durch die langsame Zugabe von 440 mg fein zerriebenem Ammoniumsulfat (AS) auf eine AS-Sättigung von 70 % gebracht. Nach ungefähr 12 Stunden bei 4°C wird das präzipitierte IFNγ durch Zentrifugation (30 Minuten bei 10000 rpm; JA 20 Rotor) als ein AS-Pellet gesammelt. Das Pellet wird in 1 ml Puffer aufgenommen (Fraktion III).

### Gelfiltration

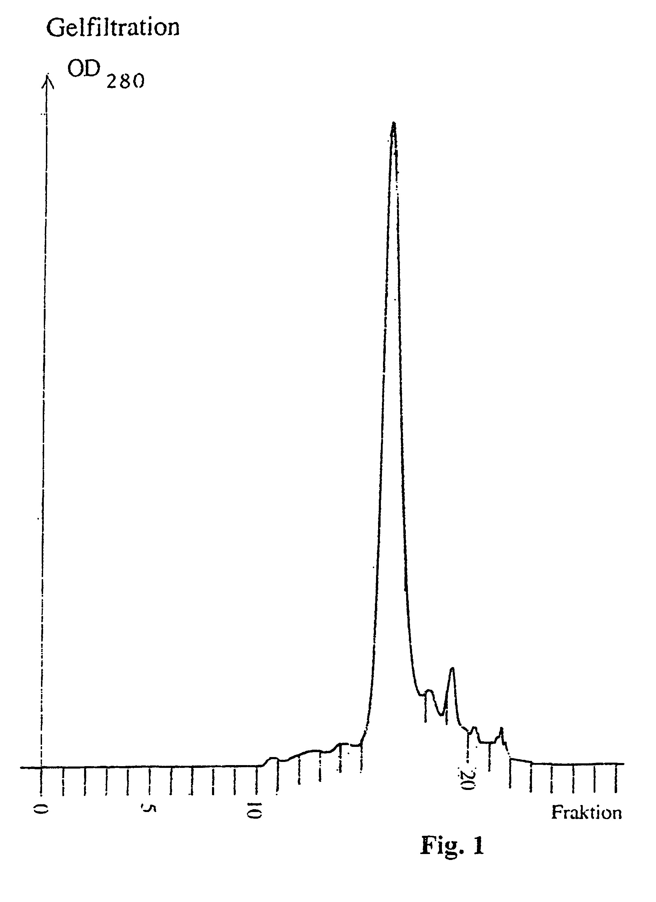

1 ml Fraktion wird sterifiltriert (0,2 µM Porengröße) und in unterschiedlicher Probengröße (200 µl - 1 ml) mit Hilfe einer TSKG 200 Säule fraktioniert. Dieser Gelfiltrationsschritt ergibt (Figur 1) eine symmetrische Verteilung des IFNγ Proteins und zeigt mit Hilfe von Eichproteinen an, daß das IFNγ als ein homodimeres Protein vorliegt.

### Charakterisierung des löslichen IFNγ

Eine SDS-Gelelektrophorese-Analyse (SDS-Gel) ergibt einen Reinheitsgrad von mehr als 95 %. Obwohl im direkten Vergleich mit dem entsprechenden IFNγ aus "Inclusionbodies" ein etwas schnelleres Wanderungsverhalten beobachtet wird, das auf eine minimale Verkürzung um wenige Aminosäuren hindeutet, kann das lösliche IFNγ mit dieser Prozedur als ein insgesamt intaktes Protein gewonnen werden. Die spezifische antivirale Aktivität des löslichen IFNγ mit 2 x 10⁷ n/mg Protein entspricht dem des IFNγ aus "Inclusionbodies".

### Vorteile des löslichen IFNγ

Der Vorteil dieses Verfahrens liegt darin, daß die Faltung des IFNγ Proteins in die räumliche Struktur in der Bakterienzelle abläuft; es gibt keinen Hinweis auf eine inhomogene Strukturpopulation für dieses Protein. Mit dem gleichen Ansatz kann auch eine Variante des IFNγ, die um 10 Aminosäuren verkürzt wurde, und eine Variante, der zwei Cysteine so eingefügt werden, daß sich eine interne Disulfidbrücke ausbilden kann, synthetisiert und hochgereinigt werden.

Die Ausbeute im Reinigungsverfahren liegt bei bis zu 50 % für alle untersuchten IFNγ Proteine und hebt sich damit von den 10 %igen Ausbeute für IFNγ ab, das aus den "Inclusionbodies" herausgelöst und in vitro im Reagenzglas in die richtige räumliche Struktur gefaltet werden muß. Aus 4 g Zellpellet können ungefähr 4 mg hochreines lösliches IFNγ gewonnen werden.

Das lösliche IFNγ mit einer Disulfidbrücke kann sowohl als ein redulziertes als auch ein oxidiertes Protein gereinigt und gehalten werden. Das entsprechende IFNγ aus "Inclusionbodies" kann nur in oxidierter Form gewonnen werden.

Es ist davon auszugehen, daß das in vitro gefaltete IFNγ strukturell inhomogen ist, das lösliche IFNγ homogen vorliegt.

Für einen klinischen Einsatz ist deshalb das lösliche, erfindungsgemäße IFNγ gar nicht oder zumindest weniger antigen als das "Inclusionbody" IFNγ.

## Patentansprüche

1. Verfahren zur Herstellung von löslichen, rekombinanten Proteinen aus E. coli, bei denen das Plasmid das vollständige Gen hinter einem trc Promotor enthält, wobei dieses Plasmid exprimiert und das erhaltene Protein isoliert und gereinigt wird,
**dadurch gekennzeichnet , daß**
a) die Zellkultur bis zu einer OD 600 nm von 0,4 bis 0,6 hochgezogen;
b) die Zellkultur über eine Zeitspanne von 15 bis 40 Minuten auf 20 bis 26°C abgekühlt;
c) die Synthese durch Zugabe von 10 bis 55 µM eines IPTG-Induktors induziert;
d) die Induktionsphase bei einer Temperatur von 20 bis 26°C über eine Zeitspanne von 3,5 bis 6,5 Stunden durchgeführt; und
e) die erhaltenen Zellpellets in einem Puffer aufgenommen in "Inclusionbodies" und einen Proteinüberstand aufgetrennt und daß das Protein ausschließlich aus dem Proteinüberstand isoliert wird.

2. Verfahren nach Anspruch 1,
**dadurch gekennzeichnet, daß** die Zellkulturen (Verfahrensschritt a)) bis zu einer OD von 0,45 bis 0,55 bei 35 bis 38°C hochgezogen werden.

3. Verfahren nach Anspruch 1 oder 2,
dadurch gekenzeichnet, daß die Zellkulturen (Verfahrensschritt b)) über eine Zeitspanne von 25 bis 35 Min. auf 24 bis 26°C abgekühlt werden.

4. Verfahren nach mindestens einem der Ansprüche 1 bis 3,
**dadurch gekennzeichnet, daß** die Synthese (Verfahrensschritt c)) durch Zugabe von 10 bis 50 µM eines Induktors (Endkonzentration) induziert wird.

5. Verfahren nach mindestens einem der Ansprüche 1 bis 4,
**dadurch gekennzeichnet, daß** die Induktionsphase (Verfahrensschritt d)) bei einer Temperatur von 24 bis 26°C über eine Zeitspanne von 3,5 bis 5,5 Std. durchgeführt wird.

6. Verfahren nach mindestens einem der Ansprüche 1 bis 5,
**dadurch gekennzeichnet, daß** als Gen das komplette Gen für IFNγ eingesetzt wird.

## Claims

1. Method of producing soluble recombinant protein from *E.coli*, in which the plasmid contains the complete gene behind a trc promoter, this plasmid being expressed and the protein obtained being isolated and purified,
**characterised in that**
a) the cell culture is grown to an OD 600 nm of 0.4 to 0.6;
b) the cell culture is cooled to 20 to 26°C over a period of 15 to 40 minutes;
c) synthesis is induced by the addition of 10 to 55 µM of an IPTG inductor;
d) the induction phase is carried out at a temperature of 20 to 26°C over a period of 3.5 to 6.5 hours; and
e) the cell pellets obtained are taken up in a buffer and separated into inclusion bodies and a protein residue and **in that** the protein is isolated solely from the protein residue.

2. Method according to claim 1, **characterised in that** the cell cultures (Method step a)) are grown to an OD of 0.45 to 0.55 at 35 to 38°C.

3. Method according to claim 1 or 2, **characterised in that** the cell cultures (Method step b)) are cooled to 24 to 26°C over a period of 25 to 35 min.

4. Method according to at least one of claims I to 3, **characterised in that** synthesis (Method step c)) is induced by the addition of 10 to 50 µM of an inductor (final concentration).

5. Method according to at least one of claims 1 to 4, **characterised in that** the induction phase (Method step d)) is carried out at a temperature of 24 to 26°C over a period of 3.5 to 5.5 hours.

6. Method according to at least one of claims 1 to 5, **characterised in that** the complete gene for IFNγ is used as the gene.

## Revendications

1. Procédé de fabrication de protéines recombinantes solubles à partir de E.coli, dans lesquelles le plasmide contient le gène complet derrière un promoteur trc, ce plasmide étant exprimé et la protéine obtenue étant isolée et purifiée,
**caractérisé en ce que**
a) la culture cellulaire est relevée de 0,4 à 0,6 jusqu'à un OD de 600 nm ;
b) la culture cellulaire est refroidie jusque 20 à 26°C en une période de temps de 15 à 40 minutes ;
c) la synthèse est induite par addition de 10 à 55 µM d'un inducteur IPTG ;
d) la phase d'induction est exécutée à une température de 20 à 26°C en une période de temps de 3,5 à 6,5 heures ;
e) les pastilles cellulaires obtenues placées dans un tampon sont séparées en "corps d'inclusions" et un excédent de protéine, et la protéine est isolée exclusivement à partir de l'excédent de protéine.

2. Procédé selon la revendication 1,
**caractérisé en ce que**
les cultures cellulaires (étape a)) sont relevées à 35 à 38°C jusqu'à un OD de 0,45 à 0,55.

3. Procédé selon la revendication 1 ou 2,
**caractérisé en ce que**
les cultures cellulaires (étape b)) sont refroidies à 24 à 26°C en une période de temps de 25 à 35 minutes.

4. Procédé selon au moins une des revendications 1 à 3,
**caractérisé en ce que**
la synthèse (étape c)) est induite par addition de 10 à 50 µM d'un inducteur (concentration finale).

5. Procédé selon au moins une des revendications 1 à 4,
**caractérisé en ce que**
la phase d'induction (étape d)) est exécutée à une température de 24 à 26°C en une période de temps de 3,5 à 5,5 heures.

6. Procédé selon au moins une des revendications 1 à 5,
**caractérisé en ce que**
comme gène on utilise le gène complet pour IFNγ.
